(19) **Europäisches Patentamt / European Patent Office / Office européen des brevets**

(11) **EP 2 425 784 B1**

(12) **EUROPEAN PATENT SPECIFICATION**

(45) Date of publication and mention
of the grant of the patent:
**18.05.2016 Bulletin 2016/20**

(21) Application number: **11176129.2**

(22) Date of filing: **01.08.2011**

(51) Int Cl.:
*A61B 8/08* (2006.01)     *G01S 15/89* (2006.01)
*G06T 7/00* (2006.01)     *A61B 8/00* (2006.01)
*G01S 7/52* (2006.01)

(54) **Providing a color Doppler mode image in an ultrasound system**

Bereitstellung eines Farbdopplermodusbilds in einem Ultraschallsystem

Fourniture d'une image en mode Doppler couleur dans un système à ultrasons

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2010 KR 20100085969**

(43) Date of publication of application:
**07.03.2012 Bulletin 2012/10**

(73) Proprietor: **Samsung Medison Co., Ltd.
Hongcheon-gun, Gangwon-do, 25108 (KR)**

(72) Inventors:
 • **Lee, Jae Keun
  135-851 Seoul (KR)**
 • **Lee, Hyeong Do
  135-851 Seoul (KR)**

 • **Lee, Yong Ho
  135-851 Seoul (KR)**

(74) Representative: **Schmid, Wolfgang
Lorenz & Kollegen
Patentanwälte Partnerschaftsgesellschaft mbB
Alte Ulmer Strasse 2
89522 Heidenheim (DE)**

(56) References cited:
 **US-A1- 2004 207 760**     **US-A1- 2005 059 892**

 • **JACINTA E BROWNE ET AL: "COLOUR DOPPLER SPATIAL RESOLUTION PERFORMANCE TESTING", ULTRASOUND, ROYAL SOCIETY OF MEDICINE PRESS, GB, vol. 15, 1 August 2007 (2007-08-01), pages 162-166, XP007919778, ISSN: 1742-271X, DOI: 10.1179/174313407X208677**

EP 2 425 784 B1

## Description

### CROSS-REFERENCE TO RELATED APPLICATION

The present application claims priority from Korean Patent Application No. 10-2010-0085969 filed on September 2, 2010.

### TECHNICAL FIELD

The present disclosure generally relates to ultrasound systems, and more particularly to providing a color Doppler mode image in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional or three-dimensional ultrasound images of internal features of an object (e.g., human organs).

The ultrasound system provides ultrasound images of various modes including a brightness mode (B mode) image representing reflection coefficients of the ultrasound signals reflected from a target object of a living body with a two-dimensional image, a Doppler mode image representing speed of a moving object with spectral Doppler by using a Doppler effect, a color Doppler mode image representing speed of a moving object with colors by using the Doppler effect, and an elastic mode image representing mechanical characteristics of tissues before and after applying a pressure thereto. Particularly, the ultrasound system transmits and receives ultrasound signals to and from the target object to thereby form Doppler signals corresponding to a region of interest (ROI), which is set on a B mode image. The ultrasound system further forms a color Doppler mode image that represents the speed of the moving object with colors based on the Doppler signals.

The ultrasound system performs a balance process to eliminate the color Doppler mode image corresponding to regions wherein a brightness value corresponding to a pixel of a brightness mode image is larger than a predetermined balance threshold value upon the color Doppler mode image. This is to remove color artifacts, which occurs by motion of regions such as blood-vessel walls, except interior spaces of the blood vessels, i.e., a lumen in which blood flows. However, this can be a problem since the conventional balance process cannot remove the color artifacts perfectly and decreases quality of the color Doppler mode image.

The US 2005/059892A1 discloses an ultrasound imaging system lateral gain control circuit operating in conjunction with an ultrasound imaging system for generating an ultrasound image of an ultrasound analysis object using an ultrasound sensing circuit for sensing an ultrasound signal returning from an ultrasound analysis object to an ultrasound transducer. The ultrasound image is defined by a first region and a second region. The first region and second region together form the complete ultrasound image. The first region associates with a region proximate to the ultrasound transducer, while the second region associates with a region distal from the ultrasound transducer. The lateral gain control circuit controls the two-dimensional ultrasound image brightness between the two regions by controlling the ultrasound signal amplification by separately controlling the first region signal amplification and the second region signal amplification.

Reference is also made to Jacinta E Browne et al: "Colour Doppler Spatial Resolution Performance Testing", Ultrasound, Royal Society of Medicine Press, GB, vol. 15, 1 August 2007 (2007-08-01), pages 162-166, XP007919778, ISSN: 1742-271X, D01: 10.1179/174313407X208677.

### SUMMARY

Embodiments for providing a color Doppler mode image in an ultrasound system are disclosed herein. In one embodiment, according to claim 1, an ultrasound system comprises: an ultrasound data acquisition unit configured to transmit and receive ultrasound signals to and from a living body to acquire first ultrasound data and second ultrasound data; a user input unit configured to receive input information corresponding to a region of interest and a balance threshold value having a predetermined brightness value; and a processing unit in communication with the ultrasound data acquisition unit and the user input unit, the processing unit being configured to form a brightness mode image and a color Doppler mode image corresponding to the region of interest based on the first and second ultrasound data, respectively, set a contrast stretching curve for securing connectivity of blood vessels of the living body based on the balance threshold value of the input information and the brightness mode image, form a mask for performing a balance process upon the color Doppler mode image based on the contrast stretching curve, and perform the balance process upon the color Doppler mode image based on the mask.

In another embodiment, there is provided a method according to claim 8 of providing a color Doppler mode image, comprising: a) forming a brightness mode image based on first ultrasound data for a living body; b) receiving input information corresponding to a region of interest and a balance threshold value having a predetermined brightness value from a user; c) forming a color Doppler mode image corresponding to the region of interest based on second ultrasound data for the living body; d) setting a contrast stretching curve for securing connectivity of blood vessels of the living body based on the balance threshold value of the input information and the brightness mode image, and forming a mask for performing a balance process upon the color Doppler mode image based on the contrast stretching curve; and e) performing the balance process upon the color Doppler mode image based on the mask.

BRIEF DESCRIPTION OF THE DRAWINGS

FIG. 1    is a block diagram showing an illustrative embodiment of an ultrasound system.
FIG. 2    is a schematic diagram showing an example of a brightness mode image and a region of interest.
FIG. 3    is a block diagram showing an illustrative embodiment of an ultrasound data acquisition unit.
FIG. 4    is a flow chart showing a process of performing a balance process upon a color Doppler mode image.
FIG. 5    is a flow chart showing a process of forming a balance mask.
FIG. 6    is a schematic diagram showing an example of an image parameter.
FIG. 7    is a schematic diagram showing another example of the image parameter.
FIG. 8    is a schematic diagram showing yet another example of the image parameter.
FIG. 9    is a schematic diagram showing still yet another example of the image parameter.

DETAILED DESCRIPTION

A detailed description may be provided with reference to the accompanying drawings. One of ordinary skill in the art may realize that the following description is illustrative only and is not in any way limiting.

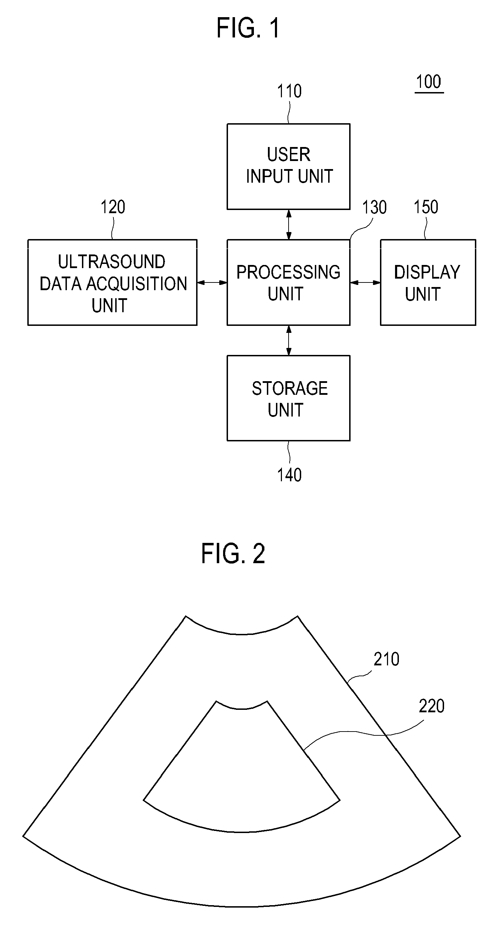

Referring to FIG. 1, an ultrasound system 100 in accordance with an illustrative embodiment is shown. As depicted therein, the ultrasound system 100 includes a user input unit 110. The user input unit 110 is configured to receive input information for a user. In one embodiment, the input information includes first input information for setting a region of interest (ROI) 220 on a brightness mode image 210 as shown in FIG. 2. The ROI 220 includes a color box for obtaining a color Doppler mode image. The input information further includes second input information for setting a threshold value having a predetermined brightness value ("balance threshold value") for performing a balance processing upon the color Doppler mode image corresponding to the ROI 220. However, it should be noted herein that the input information may not be limited thereto. The user input unit 110 includes a control panel, a track ball, a mouse, a keyboard and the like.

The ultrasound system 100 further includes an ultrasound data acquisition unit 120. The ultrasound data acquisition unit 120 is configured to transmit and receive ultrasound signals to and from a living body and output ultrasound data. The living body includes a plurality of target objects (e.g., blood vessels, heart, etc.).

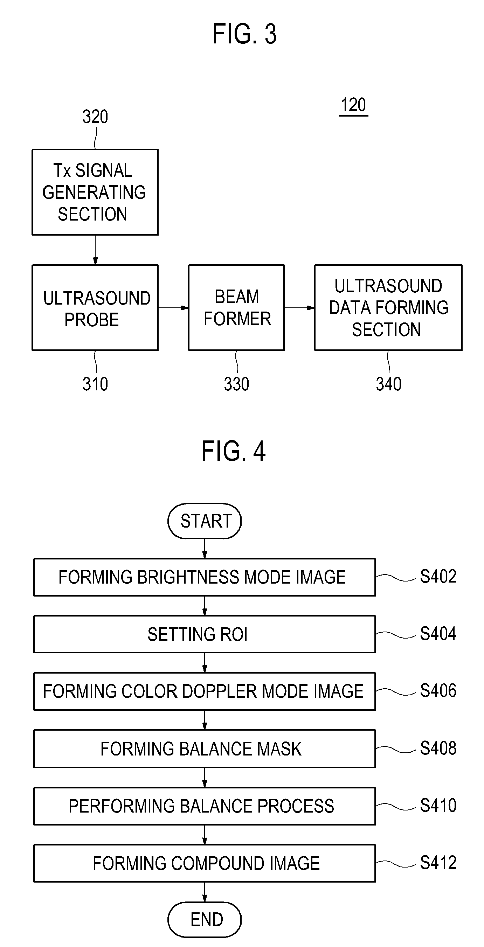

FIG. 3 is a block diagram showing an illustrative embodiment of the ultrasound data acquisition unit 120. Referring to FIG. 3, the ultrasound data acquisition unit 120 includes an ultrasound probe 310. The ultrasound probe 310 includes a plurality of elements (not shown) for reciprocally converting between ultrasound signals and electrical signals. The ultrasound probe 310 is configured to transmit ultrasound signals to the living body. The ultrasound probe 310 is further configured to receive ultrasound signals (i.e., ultrasound echo signals) from the living body to output received signals. The received signals are analog signals. The ultrasound probe 310 includes a convex probe, a linear probe and the like.

The ultrasound data acquisition unit 120 further includes a transmit (Tx) signal generating section 320. The Tx signal generating section 320 is configured to control the transmission of the ultrasound signals. The Tx signal generating section 320 is further configured to generate electrical signals ("Tx signals") for obtaining at least one ultrasound image in consideration of the elements and focal points.

In one embodiment, the Tx signal generating section 320 is configured to generate first Tx signals for obtaining the brightness mode image. Thus, the ultrasound probe 310 is configured to convert the first Tx signals provided from the Tx signal generating section 320 into the ultrasound signals, transmit the ultrasound signals to the living body and receive the ultrasound echo signals from the living body to thereby output first received signals. The Tx signal generating section 320 is further configured to generate second Tx signals for obtaining the color Doppler mode image based on a predetermined ensemble number. The ensemble number represents the number of transmitting and receiving ultrasound signals in order to obtain Doppler signals corresponding to each of the scan-lines. Thus, the ultrasound probe 310 is configured to convert the second Tx signals provided from the Tx signal generating section 320 into the ultrasound signals, transmit the ultrasound signals to the living body and receive the ultrasound echo signals from the living body to thereby output second received signals.

The ultrasound data acquisition unit 120 further includes a beam former 330. The beam former 330 is configured to convert the received signals provided from the ultrasound probe 310 into digital signals. The beam former 330 is further configured to apply delays to the digital signals in consideration of the elements and the focal points to thereby output digital receive-focused signals.

In one embodiment, the beam former 330 is configured to convert the first received signals provided the ultrasound probe 310 into first digital signals. The beam former 330 is further configured to apply delays to the first digital signals in consideration of the elements and the focal points to output first digital receive-focused signals. Also, the beam former 330 is configured to convert the second received signals provided from the ultrasound probe 310 into second digital signals. The beam former 330 is further configured to apply delays to the second digital signals in consideration of the elements and the focal points to output second digital receive-focused signals.

The ultrasound data acquisition unit 120 further includes an ultrasound data forming section 340. The ultrasound data forming section 340 is configured to form ultrasound data based on the digital receive-focused signals provided from the beam former 330. The ultrasound data forming section 340 is further configured to perform signal processing (e.g., gain control, etc.) upon the digital receive-focused signals.

In one embodiment, the ultrasound data forming section 340 is configured to form first ultrasound data based on the first digital receive-focused signals provided from the beam former 330. The first ultrasound data includes radio frequency data. However, it should be noted herein that the first ultrasound data may not be limited thereto. The ultrasound data forming section 340 is further configured to form second ultrasound data based on the second digital receive-focused signals provided from the beam former 330. The second ultrasound data includes in-phase/quadrature (IQ) data. However, it should be noted herein that the second ultrasound data may not be limited thereto.

Although it is described above that the ultrasound data acquisition unit 120 is configured to acquire the ultrasound data by transmitting and receiving the ultrasound signals to and from the living body, the ultrasound data acquisition unit 120 is further configured to acquire the ultrasound data from an external or internal storage unit (not shown) connected to the ultrasound system 100.

Referring back to FIG. 1, the ultrasound system 100 further includes a processing unit 130 in communication with the user input unit 110 and the ultrasound data acquisition unit 120. The processing unit 130 includes a central processing unit, a microprocessor or a graphic processing unit. However, it should be noted herein that the processing unit 130 may not be limited thereto.

FIG. 4 is a flow chart showing a process of performing the balance process upon the color Doppler mode image. The processing unit 130 is configured to form the brightness mode image based on the first ultrasound data provided from the ultrasound data acquisition unit 120 at step S402 in FIG. 4. The brightness mode image is displayed on a display unit 150. Thus, the user sets the ROI on the brightness mode image displayed on the display unit 150 by using the user input unit 110.

The processing unit 130 is configured to set the ROI on the brightness mode image based on the input information (i.e., first input information) provided from the user input unit 110 at step S404 in FIG. 4. Thus, the ultrasound data acquisition unit 120 transmits and receives the ultrasound data to and from the living body to thereby acquire the second ultrasound data corresponding to the ROI.

The processing unit 130 is configured to form the color Doppler mode image based on the second ultrasound data provided from the ultrasound data acquisition unit 120 at step S406 in FIG. 4. The methods of the color Doppler mode image are well known in the art. Thus, they have not been described in detail so as not to unnecessarily obscure the present invention.

The processing unit 130 is configured to form a mask ("balance mask") for performing the balance process upon the color Doppler mode image based on the input information (i.e., second input information) provided from the user input unit 110 and the brightness mode image at step S408 in FIG. 4.

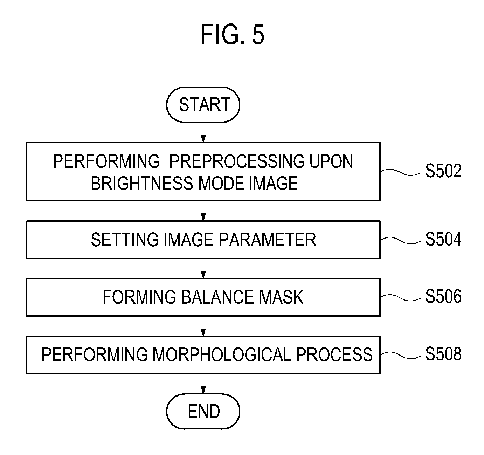

FIG. 5 is a flow chart showing a process of forming the balance mask. The processing unit 130 is configured to perform a preprocessing upon the brightness mode image to eliminate unnecessary image data at step S502 in FIG. 5. The preprocessing includes a noise elimination process by using an average filter. However, it should be noted herein that the preprocessing may not be limited thereto.

The processing unit 130 is configured to set an image parameter based on the input information (i.e., second input information) provided from the user input unit 110 and the brightness mode image at step S504 in FIG. 5. The image parameter is a parameter for forming the balance mask. That is, the image parameter is a parameter for securing connectivity of regions (e.g., blood vessels, tissues, etc.) except the inside space of the blood vessels, i.e., a lumen in which the blood flows.

In one embodiment, the processing unit 130 is configured to calculate a mean brightness value ("global mean brightness value") of pixels corresponding to the ROI of the brightness mode image based on brightness values corresponding to the pixels.

The processing unit 130 is further configured to calculate a pivot point value of a contrast stretching curve for controlling brightness levels based on the second input information (i.e., balance threshold value) provided from the user input unit 110 and the global mean brightness value. The processing unit 130 calculates the pivot point value using equation 1 provided below.

$$PPV = (V_{GM} + BTV)/2 \times V_{PPT} \qquad (1)$$

In the equation, PPV represents the pivot point value, $V_{GM}$ represents the global mean brightness value, BTV represents the balance threshold value and $V_{PPT}$ represents a pivot point threshold value. The pivot point threshold value is a predetermined value in consideration of an application and type of the target object. For example, the pivot point threshold value is 0.8 to 1.2.

The processing unit 130 is configured to calculate a gradient ("curvature") of the contrast stretching curve based on the second input information (i.e., balance threshold value) and the global mean brightness value. For example, the processing unit 130 detects a maximum brightness value from the brightness mode image. The processing unit 130 further calculates a difference between a predetermined brightness value and the maximum brightness value. The processing unit 130 further sets the difference as the gradient of the contrast stretching curve. The methods of calculating the gradient are well known in the art. For example, the processing unit 130 calculates the gradient using equation 2 provided below.

$$w = \alpha \cdot x^{d} \qquad (2)$$

In the equation, w represents the gradient, a represents a weight value, which is a predetermined value, x represents a constant value, and d represent a different value between the mean brightness value (i.e., global mean brightness value or local mean brightness value) and the balance threshold value.

As the difference between the global mean brightness value and the balance threshold value gets larger, the gradient becomes larger. As the difference between the global mean brightness value and the balance threshold value gets smaller, the gradient becomes smaller. However, it should be noted herein that the sign may not be limited thereto.

The processing unit 130 is configured to set a sign of the contrast stretching curve based on the second input information (i.e., balance threshold value) and the pivot point value. The sign includes "+" and "-." However, it should be noted herein that the sign may not be limited thereto. For example, the processing unit 130 compares the balance threshold value with the pivot point value. If the balance value is equal to or larger than the pivot point value, then the processing unit 130 sets the sign as "+." If the balance threshold value is smaller than the pivot point value, then the processing unit 130 sets the sign as "-."

The processing unit 130 is configured to set the image parameter based on the second input information, the global mean brightness value, the pivot point value, the gradient and the sign.

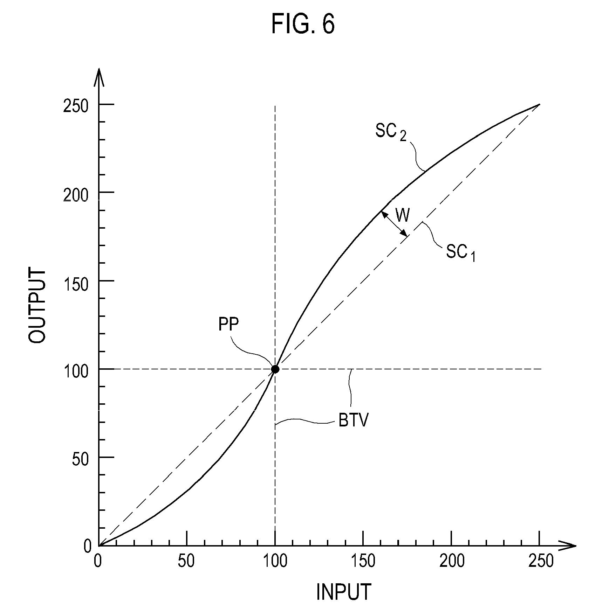

As one example, when the balance threshold value is 100, the global mean brightness value is 100 and the pivot point threshold value is 1.0, the processing unit 130 sets a first contrast stretching curve $SC_1$ based on the balance threshold value BTV as shown in FIG. 6. The processing unit 130 further calculates the pivot point value ("100") by applying the balance threshold value, the global mean brightness value and the pivot threshold value to equation 1. The processing unit 130 further calculates a difference between the balance brightness value and the global mean brightness value. The processing unit 130 further calculates the gradient W of the first contrast stretching curve $SC_1$ based on the calculated difference. The processing unit 130 further compares the balance threshold value with the pivot point value, and determines that the balance threshold value is equal to the pivot point value. The processing unit 130 further sets the sign of the first contrast stretching curve $SC_1$ as "+" based on the determining result. The processing unit 130 further sets a second contrast stretching curve $SC_2$ for setting the brightness values of regions corresponding to pixels that the brightness value is equal to or larger than 100 as a predetermined value (e.g., 255) based on the balance threshold value, the global mean brightness value, the pivot point value, the gradient and the sign, as shown in FIG. 6. The processing unit 130 further sets the second contrast stretching curve $SC_2$ as the image parameter for forming the balance mask. In FIG. 6, the reference numeral PP represents a pivot point corresponding to the pivot point value. The image parameter shown in FIG. 6 is used to form the second contrast stretching curve $SC_2$, which controls the brightness values of the brightness mode image. That is, the image parameter is used to decrease the brightness values under the pivot point value and increase the brightness values above the pivot point value.

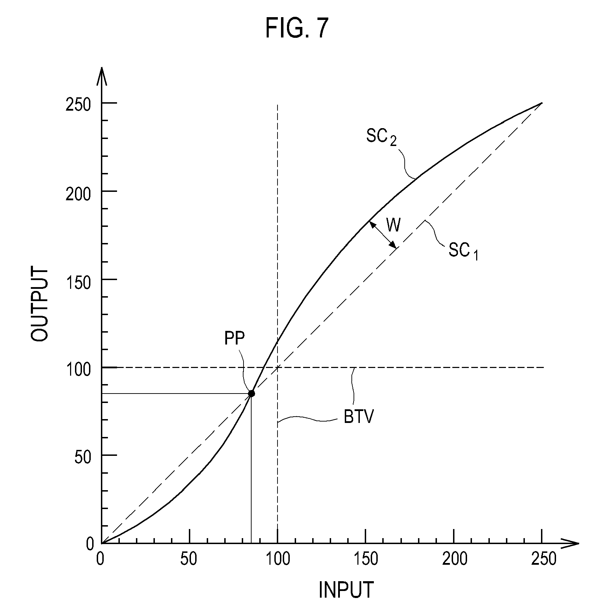

As another example, when the balance threshold value is 100, the global mean brightness value is 100 and the pivot point threshold value is 0.85, the processing unit 130 sets the first contrast stretching curve $SC_1$ based on the

balance threshold value BTV as shown in FIG. 7. The processing unit 130 further calculates the pivot point value (i.e., 85) by applying the balance threshold value (i.e., 100), the global mean brightness value (i.e., 100) and the pivot point threshold value (i.e., 0.85) to equation 1. The processing unit 130 further calculates a difference between the balance threshold value and the global mean brightness value. The processing unit 130 further calculates the gradient W of the first contrast stretching curve $SC_1$ based on the calculated difference as shown in FIG. 7. The processing unit 130 further compares the balance threshold value with the pivot point value, and determines that the balance threshold value is larger than the pivot point value. The processing unit 130 further sets the sign of the first contrast stretching curve $SC_1$ as "+" based on the determining result.

The processing unit 130 further sets the second contrast stretching curve $SC_2$ for setting the brightness values of regions corresponding to pixels that the brightness value is equal to or larger than 85 as a predetermined value (e.g., 255) based on the balance threshold value, the global mean brightness value, the pivot point value, the gradient and the sign, as shown in FIG. 7. The processing unit 130 further sets the second contrast stretching curve $SC_2$ as the image parameter for forming the balance mask. In FIG. 7, the reference numeral PP represents the pivot point corresponding to the pivot point value.

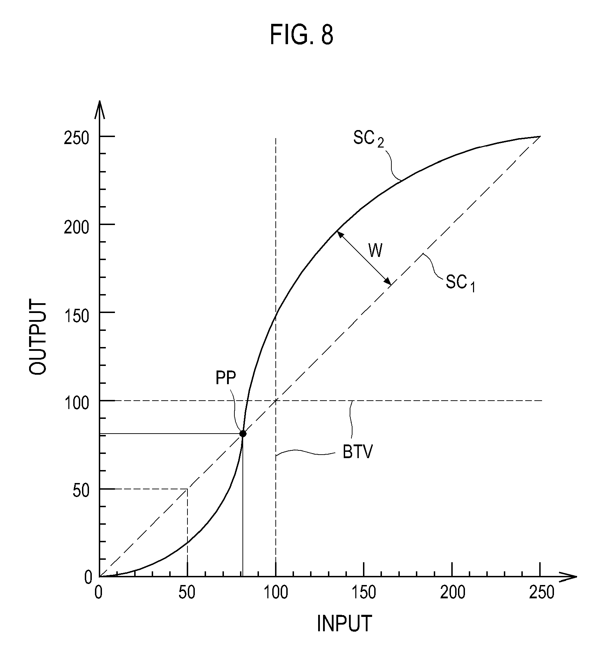

As yet another example, when the balance threshold value is 100, the global mean bright value is 50 and the pivot threshold value is 1.1, the processing unit 130 sets the first contrast stretching curve $SC_1$ based on the balance threshold value BTV as shown in FIG. 8. The processing unit 130 further calculates the pivot point value (i.e., 82.5) by applying the balance threshold value (i.e., 100), the global mean brightness value (i.e., 50) and the pivot point threshold value (i.e., 1.1) to equation 1. The processing unit 130 further calculates a difference between the balance brightness value and the global mean brightness value. The processing unit 130 further calculates the gradient W of the first contrast stretching curve $SC_1$ based on the calculated difference as shown in FIG. 8. Also, the processing unit 130 compares the balance threshold value with the pivot point value, and determines that the balance threshold value is larger than the pivot point value. The processing unit 130 further sets the sign of the first contrast stretching curve $SC_1$ as "+" based on the determining result. The processing unit 130 additionally sets the second contrast stretching curve $SC_2$ for setting the brightness values of regions corresponding to pixels wherein the brightness value is equal to or larger than 82.5 as a predetermined value (e.g., 255) based on the balance threshold value, the global mean brightness value, the pivot point value, the gradient and the sign, as shown in FIG. 8. The processing unit 130 further sets the second contrast stretching curve $SC_2$ as the image parameter for forming the balance mask. In FIG. 8, the reference numeral PP represents the pivot point corresponding to the pivot point value.

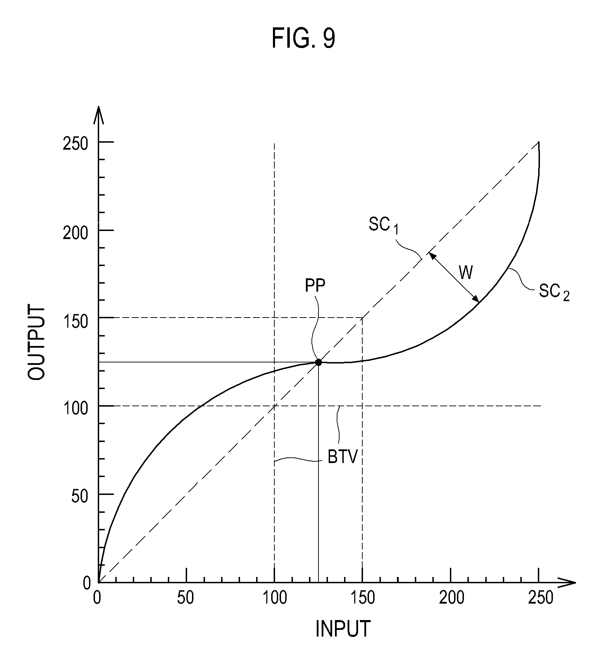

As yet another example, when the balance threshold value is 100, the global mean brightness value is 150 and the pivot point threshold value is 1.0, the processing unit 130 sets the first contrast stretching curve $SC_1$ based on the balance threshold value BTV as shown in FIG. 9. The processing unit 130 further calculates the pivot point value (i.e., 125) by applying the balance threshold value (i.e. 100), the global mean brightness value (i.e., 150) and the pivot point threshold value (i.e., 1.0) to equation 1. Also, the processing unit 130 calculates a difference between the balance threshold value and the global mean brightness value. The processing unit 130 further calculates the gradient W of the first contrast stretching curve $SC_1$ based on the calculated difference as shown in FIG. 9. The processing unit 130 further compares the balance threshold value with the pivot point value, and determines that the balance threshold value is smaller than the pivot point value. The processing unit 130 sets the sign of the first contrast stretching curve $SC_1$ as "-" based on the determining result. Also, the processing unit 130 sets the second contrast stretching curve $SC_2$ for setting the brightness values of regions corresponding to pixels wherein the brightness value is equal to or larger than 125 as a predetermined value (e.g., 255) based on the balance threshold value, the global mean brightness value, the pivot point value, the gradient and the sign, as shown in FIG. 9. The processing unit 130 further sets the second contrast stretching curve $SC_2$ as the image parameter for forming the balance mask. In FIG. 9, the reference numeral PP represents the pivot point corresponding to the pivot point value.

In another embodiment, the processing unit 130 is configured to calculate the global mean brightness value of pixels corresponding to the ROI of the brightness mode image based on the brightness values corresponding to the pixels. The processing unit 130 is further configured to divide the ROI into a plurality of regions. Each of the regions has at least one pixel. The processing unit 130 is further configured to calculate a mean brightness value ("local mean brightness value") of the at least one pixel corresponding to each of the regions. The processing unit 130 is further configured to calculate a mean brightness value of the global mean brightness value and the local mean brightness value. Also, the processing unit 130 is configured to set the image parameter based on the mean brightness value and the second input information provided from the user input unit 110, as mentioned above.

In yet another embodiment, the processing unit 130 is configured to divide the ROI into a plurality of regions. Each of the regions has at least one pixel. The processing unit 130 is further configured to calculate the local mean brightness value of the at least one pixel corresponding to each of the regions based on the brightness value of the at least one pixel. The processing unit 130 is further configured to calculate the pivot point value based on the local mean brightness value and the second input information (i.e., balance threshold value) provided from the user input unit 110. The processing unit 130 calculates the pivot point value as equation 3 provided below.

$$PPV = (V_{LM} + BTV)/2 \times V_{PPT} \qquad\qquad (3)$$

In the equation, PPV represents the pivot point value, $V_{LM}$ represents the local mean brightness value, BTV represents the balance threshold value and $V_{PPT}$ represents the pivot point threshold value.

The processing unit 130 is configured to calculate the gradient of the contrast stretching curve based on the second input information (i.e., balance threshold value) and the local mean brightness value for each of the regions, as mentioned above. The processing unit 130 is further configured to set the sign of the contrast stretching curve based on the second input information (i.e., balance brightness value) and the pivot point value, as mentioned above.

Also, the processing unit 130 is configured to set the image parameter based on the second input information, the local mean brightness value, the pivot point value, the gradient and the sign, as mentioned above.

Referring back to FIG. 5, the processing unit 130 is configured to form the balance mask based on the image parameter at step S506 in FIG. 5. In one embodiment, the processing unit 130 performs an image process upon the brightness mode image based on the image parameter for setting the brightness values of regions corresponding to pixels wherein the brightness value is equal to or larger than the pivot point value as a predetermined value (e.g., 255) to form the balance mask.

The processing unit 130 is configured to perform a morphological process for filling up empty space or enhancing connectivity upon the balance mask at step S508 in FIG. 5. The morphological process is carried out by sequentially performing dilation and erosion. That is, the balance mask is removed as many as the predetermined number of pixels and then contracted (erosion).

Although it is described above that the processing unit 130 uses the dilation and erosion as the morphological process, the processing unit 130 uses opening and closing as the morphological process.

Referring back to FIG. 4, the processing unit 130 is configured to perform the balance process upon the color Doppler mode image by using the balance mask at step S410 in FIG. 4.

The processing unit 130 is configured to compound the brightness mode image and the balance-processed color Doppler mode image to form compound image at step S412 in FIG. 4. The methods of compounding the brightness mode image and the color Doppler mode image are well known in the art. Thus, they have not been described in detail so as not to unnecessarily obscure the present invention.

Referring back to FIG. 1, the ultrasound system 100 includes a storage unit 140. The storage unit 140 stores the input information (i.e., first input information and second input information) received by the user input unit 110. The storage unit 140 further stores the ultrasound data (i.e., first ultrasound data and second ultrasound data) acquired by the ultrasound data acquisition unit 120. The storage unit 140 further stores the brightness mode image formed by the processing unit 130. The storage unit 140 further stores the color Doppler mode image formed by the processing unit 130.

The ultrasound system 100 includes the display unit 150. The display unit 150 displays the brightness mode image formed by the processing unit 130. The display unit 150 further displays the compound image formed by the processing unit 130. The display unit 150 further displays the color Doppler mode image formed by the processing unit 130.

**Claims**

1. An ultrasound system (100), comprising:

   an ultrasound data acquisition unit (120) configured to transmit and receive ultrasound signals to and from a living body to acquire first ultrasound data and second ultrasound data;
   a user input unit (110) configured to receive input information corresponding to a region of interest and a balance threshold value having a predetermined brightness value; and
   a processing unit (130) in communication with the ultrasound data acquisition unit (120) and the user input unit (110), the processing unit (130) being configured to form a brightness mode image and a color Doppler mode image corresponding to the region of interest based on the first and second ultrasound data, respectively, set a contrast stretching curve for securing connectivity of blood vessels of the living body based on the balance threshold value of the input information and the brightness mode image, form a mask for performing a balance process upon the color Doppler mode image based on the contrast stretching curve, and perform the balance process upon the color Doppler mode image based on the mask.

2. The ultrasound system (100) of Claim 1, wherein the processing unit is configured to:

calculate a mean brightness value of pixels corresponding to the region of interest of the brightness mode image;
calculate a pivot point value of the contrast stretching curve and a gradient of the contrast stretching curve based on the mean brightness value and the balance threshold value, wherein the contrast stretching curve controls brightness levels of the brightness mode image;
set a sign of the contrast stretching curve based on the balance threshold value and the pivot point value; and
set the contrast stretching curve based on the mean brightness value, the balance threshold value, the pivot point value, the gradient and the sign.

3. The ultrasound system (100) of Claim 2, wherein the processing unit (130) is configured to calculate the pivot point value as a following equation:

$$PPV = (V_{GB} + BTV)/2 \times V_{PPT}$$

wherein PPV is the pivot point value, $V_{GB}$ is the mean brightness value, BTV is the balance threshold value, and $V_{PPT}$ is a pivot point threshold value.

4. The ultrasound system (100) of Claim 1, wherein the processing unit (130) is configured to:

calculate a first mean brightness value of pixels corresponding to the region of interest of the brightness mode image;
divide the region of interest into a plurality of regions having a predetermined size;
calculate a second mean brightness value of pixels corresponding to each of the regions;
calculate a mean brightness value of the first mean brightness value and the second mean brightness value for each of the regions;
calculate a pivot point value of the contrast stretching curve and a gradient of the contrast stretching curve based on the mean brightness value and the balance threshold value, wherein the contrast stretching curve controls brightness levels of the brightness mode image;
set a sign of the contrast stretching curve based on the balance threshold value and the pivot point value; and
set the contrast stretching curve based on the mean brightness value, the balance threshold value, the pivot point value, the gradient and the sign.

5. The ultrasound system (100) of Claim 1, wherein the processing unit (130) is configured to:

divide the region of interest into a plurality of regions having a predetermined size;
calculate a mean brightness value of pixels corresponding to each of the regions;
calculate a pivot point value of the contrast stretching curve and a gradient of the contrast stretching curve based on the mean brightness value and the balance threshold value, wherein the contrast stretching curve controls brightness levels of the brightness mode image;
set a sign of the contrast stretching curve based on the balance threshold value and the pivot point value; and
set the contrast stretching curve based on the mean brightness value, the balance threshold value, the pivot point value, the gradient and the sign.

6. The ultrasound system (100) of Claim 5, wherein the processing unit (130) is configured to calculate the pivot point value as a following equation:

$$PPV = (V_{LM} + BTV)/2 \times V_{PPT}$$

wherein PPV is the pivot point value, $V_{LM}$ is the mean brightness value, BTV represents the balance threshold value and $V_{PPT}$ represents the pivot point threshold value.

7. The ultrasound system of any of Claims 1 - 6, wherein the processing unit (130) is further configured to:

perform a preprocessing upon the brightness mode image to eliminate unnecessary image data; and
perform a morphological process upon the mask to fill up empty space or enhance connectivity upon the mask.

8. A method of providing a color Doppler mode image, comprising:

a) forming a brightness mode image based on first ultrasound data for a living body;
b) receiving input information corresponding to a region of interest and a balance threshold value having a predetermined brightness value from a user;
c) forming a color Doppler mode image corresponding to the region of interest based on second ultrasound data for the living body;
d) setting a contrast stretching curve for securing connectivity of blood vessels of the living body based on the balance threshold value of the input information and the brightness mode image, and forming a mask for performing a balance process upon the color Doppler mode image based on the contrast stretching curve; and
e) performing the balance process upon the color Doppler mode image based on the mask.

9. The method of Claim 8, wherein the step d) includes:

calculating a mean brightness value of pixels corresponding to the region of interest of the brightness mode image;
calculating a pivot point value of the contrast stretching curve and a gradient of the contrast stretching curve based on the mean brightness value and the balance threshold value, wherein the contrast stretching curve controls brightness levels of the brightness mode image;
setting a sign of the contrast stretching curve based on the balance threshold value and the pivot point value;
setting the contrast stretching curve for forming the mask based on the mean brightness value, the balance threshold value, the pivot point value, the gradient and the sign; and
forming the mask based on the contrast stretching curve.

10. The method of Claim 9, wherein the pivot point value is calculated as a following equation:

$$PPV = (V_{GB} + BTV)/2 \times V_{PPT}$$

wherein PPV is the pivot point value, $V_{GB}$ is the mean brightness value, BTV is the balance threshold value, and $V_{PPT}$ is a pivot point threshold value.

11. The method of Claim 9, wherein the step d) includes:

calculating a first mean brightness value of pixels corresponding to the region of interest of the brightness mode image;
dividing the region of interest into a plurality of regions having a predetermined size;
calculating a second mean brightness value of pixels corresponding to each of the regions;
calculating a mean brightness value of the first mean brightness value and the second mean brightness value for each of the region;
calculating a pivot point value of the contrast stretching curve and a gradient of the contrast stretching curve based on the mean brightness value and the balance threshold value, wherein the contrast stretching curve controls brightness levels of the brightness mode image;
setting a sign of the contrast stretching curve based on the balance threshold value and the pivot point value;
setting the contrast stretching curve for forming the mask based on the mean brightness value, the balance threshold value, the pivot point value, the gradient and the sign; and
forming the mask based on the contrast stretching curve.

12. The method of Claim 9, wherein the step d) includes:

dividing the region of interest into a plurality of regions having a predetermined size;
calculating a mean brightness value of pixels corresponding to each of the regions;
calculating a pivot point value of the contrast stretching curve and a gradient of the contrast stretching curve based on the mean brightness value and the balance threshold value, wherein the contrast stretching curve controls brightness levels of the brightness mode image;
setting a sign of the contrast stretching curve based on the balance threshold value and the pivot point value;
setting the contrast stretching curve for forming the mask based on the mean brightness value, the threshold value, the pivot point value, the gradient and the sign; and
forming the mask based on the contrast stretching curve.

13. The method of the Claim 12, wherein the pivot point value is calculated as following equation:

$$PPV = (V_{LM}+BTV)/2 \times V_{PPT}$$

wherein PPV is the pivot point value, $V_{LM}$ is the mean brightness value, BTV represents the balance threshold value and $V_{PPT}$ represents the pivot point threshold value.

14. The method of any of Claims 8 to 13, wherein the step d) further includes:

performing a preprocessing upon the brightness mode image to eliminate unnecessary image data; and
performing a morphological process upon the mask to fill up empty space or
enhance connectivity upon the mask.

## Patentansprüche

1. Ultraschallsystem (100), welches Folgendes aufweist:

eine Ultraschalldatenerlangungseinheit (120), die dafür vorgesehen ist, Ultraschallsignale zu und von einem lebenden Körper zu übertragen und zu empfangen, um erste Ultraschalldaten und zweite Ultraschalldaten zu erlangen;
eine Benutzereingabeeinheit (110), die dafür vorgesehen ist, einem interessierenden Bereich entsprechende Eingabeinformationen und einen Balance-grenzwert, der einen bestimmten Helligkeitswert aufweist, zu empfangen; und
eine Verarbeitungseinheit (130) in Verbindung mit der Ultraschalldatenerlangungseinheit (120) und der Benutzereingabeeinheit (110), wobei die Verarbeitungseinheit (130) dafür vorgesehen ist, ein Helligkeitsmodusbild und ein Farb-Doppler-Modusbild entsprechend dem interessierenden Bereich basierend auf den jeweiligen ersten und zweiten Ultraschalldaten zu erzeugen, eine Kontrastdehnungskurve zum Sicherstellen der Konnektivität von Blutgefäßen des lebenden Körpers basierend auf dem Balancegrenzwert der Eingabeinformation und dem Helligkeitsmodusbild festzulegen, eine Maske zur Durchführung eines Balanceprozesses an dem Farb-Doppler-Modusbild basierend auf der Kontrastdehnungskurve zu erzeugen und den Balanceprozess an dem Farb-Doppler-Modusbild basierend auf der Maske durchzuführen.

2. Ultraschallsystem (100) nach Anspruch 1, wobei die Verarbeitungseinheit für Folgendes vorgesehen ist:

Berechnen eines durchschnittlichen Helligkeitswerts von dem interessierenden Bereich entsprechenden Pixeln des Helligkeitsmodusbilds;
Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;
Festlegen eines Vorzeichens der Kontrastdehnungskurve basierend auf dem Balancegrenzwert und dem Drehpunktwert; und
Festlegen der Kontrastdehnungskurve basierend auf dem durchschnittlichen Helligkeitswert, dem Balancegrenzwert, dem Drehpunktwert, dem Gradienten und dem Vorzeichen.

3. Ultraschallsystem (100) nach Anspruch 2, wobei die Verarbeitungseinheit (130) dafür vorgesehen ist, den Drehpunktwert aus einer nachfolgend angegebenen Gleichung zu berechnen:

$$PPV = (V_{GB}+BTV)/2 \times V_{PPT}$$

wobei PPV der Drehpunktwert ist, $V_{GB}$ der durchschnittliche Helligkeitswert ist, BTV der Balancegrenzwert ist und $V_{PPT}$ ein Drehpunktgrenzwert ist.

4.  Ultraschallsystem (100) nach Anspruch 1, wobei die Verarbeitungseinheit (130) für Folgendes vorgesehen ist:

    Berechnen eines ersten durchschnittlichen Helligkeitswerts von dem interessierenden Bereich entsprechenden Pixeln des Hellligkeitsmodusbilds;
    Aufteilen des interessierenden Bereichs in eine Vielzahl von Bereichen, die eine vorbestimmte Größe aufweisen;
    Berechnen eines zweiten durchschnittlichen Helligkeitswerts von jedem der Bereiche entsprechenden Pixeln;
    Berechnen eines durchschnittlichen Helligkeitswerts des ersten durchschnittlichen Helligkeitswerts und des zweiten durchschnittlichen Helligkeitswerts für jeden der Bereiche;
    Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;
    Festlegen eines Vorzeichens der Kontrastdehnungskurve basierend auf dem Balancegrenzwert und dem Drehpunktwert; und
    Festlegen der Kontrastdehnungskurve basierend auf dem durchschnittlichen Helligkeitswert, dem Balancegrenzwert, dem Drehpunktwert, dem Gradienten und dem Vorzeichen.

5.  Ultraschallsystem (100) nach Anspruch 1, wobei die Verarbeitungseinheit (130) für Folgendes vorgesehen ist:

    Aufteilen des interessierenden Bereichs in eine Vielzahl von Bereichen, die eine bestimmte Größe aufweisen;
    Berechnen eines durchschnittlichen Helligkeitswerts von jedem der Bereiche entsprechenden Pixeln;
    Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;
    Festlegen eines Vorzeichens der Kontrastdehnungskurve basierend auf dem Balancegrenzwert und dem Drehpunktwert; und
    Festlegen der Kontrastdehnungskurve basierend auf dem durchschnittlichen Helligkeitswert, dem Balancegrenzwert, dem Drehpunktwert, dem Gradienten und dem Vorzeichen.

6.  Ultraschallsystem (100) nach Anspruch 5, wobei die Verarbeitungseinheit (130) dafür vorgesehen ist, den Drehpunktwert aus einer nachfolgend angegebenen Gleichung zu berechnen:

$$PPV = (V_{LM}+BTV)/2 \times V_{PPT}$$

wobei PPV der Drehpunktwert ist, $V_{LM}$ der durchschnittliche Helligkeitswert ist, BTV den Balancegrenzwert repräsentiert und $V_{PPT}$ den Drehpunktgrenzwert repräsentiert.

7.  Ultraschallsystem nach einem der Ansprüche 1 - 6, wobei die Verarbeitungseinheit (130) des Weiteren für Folgendes vorgesehen ist:

    Durchführen einer Vorverarbeitung an dem Helligkeitsmodusbild, um unnötige Bilddaten zu entfernen; und
    Durchführen eines morphologischen Prozesses an der Maske, um Leerräume aufzufüllen oder die Konnektivität an der Maske zu verbessern.

8.  Verfahren zur Bereitstellung eines Farb-Doppler-Modusbilds, welches Folgendes aufweist:

    a) Erzeugen eines Helligkeitsmodusbilds basierend auf ersten Ultraschalldaten für einen lebenden Körper;
    b) Empfangen von Eingabeinformationen, die einem interessierenden Bereich und einem Balancegrenzwert mit einem bestimmten Helligkeitswert entsprechen, von einem Benutzer;
    c) Erzeugen eines dem interessierenden Bereich entsprechenden Farb-Doppler-Modusbilds basierend auf zweiten Ultraschalldaten für den lebenden Körper;
    d) Festlegen einer Kontrastdehnungskurve zum Sicherstellen von Konnektivität von Blutgefäßen des lebenden Körpers basierend auf dem Balancegrenzwert der Eingabeinformation und dem Helligkeitsmodusbild und Erzeugen einer Maske zur Durchführung eines Balanceprozesses an dem Farb-Doppler-Modusbild basierend auf der Kontrastdehnungskurve; und
    e) Durchführen des Balanceprozesses an dem Farb-Doppler-Modusbild basierend auf der Maske.

9.  Verfahren nach Anspruch 8, wobei der Schritt d) Folgendes aufweist:

Berechnen eines durchschnittlichen Helligkeitswerts von dem interessierenden Bereich entsprechenden Pixeln des Helligkeitsmodusbilds;

Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;

Festlegen eines Vorzeichens der Kontrastdehnungskurve basierend auf dem Balancegrenzwert und dem Drehpunktwert;

Festlegen der Kontrastdehnungskurve basierend auf dem durchschnittlichen Helligkeitswert, dem Balance-grenzwert, dem Drehpunktwert, dem Gradienten und dem Vorzeichen; und

Erzeugen der Maske basierend auf der Kontrastdehnungskurve.

10. Verfahren nach Anspruch 9, wobei der Drehpunktwert aus einer nachfolgend angegebenen Gleichung berechnet wird:

$$PPV = (V_{GB}+BTV)/2 \times V_{PPT}$$

wobei PPV der Drehpunktwert ist, $V_{GB}$ der durchschnittliche Helligkeitswert ist, BTV der Balancegrenzwert ist und $V_{PPT}$ ein Drehpunktgrenzwert ist.

11. Verfahren nach Anspruch 9, wobei der Schritt d) Folgendes aufweist:

Berechnen eines ersten durchschnittlichen Helligkeitswerts von dem interessierenden Bereich entsprechenden Pixeln des Helligkeitsmodusbilds;

Aufteilen des interessierenden Bereichs in eine Vielzahl von Bereichen, die eine vorbestimmte Größe aufweisen;

Berechnen eines zweiten durchschnittlichen Helligkeitswerts von jedem der Bereiche entsprechenden Pixeln;

Berechnen eines durchschnittlichen Helligkeitswerts des ersten durchschnittlichen Helligkeitswerts und des zweiten durchschnittlichen Helligkeitswerts für jeden der Bereiche;

Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;

Festlegen eines Vorzeichens der Kontrastdehnungskurve basierend auf dem Balancegrenzwert und dem Drehpunktwert;

Festlegen der Kontrastdehnungskurve basierend auf dem durchschnittlichen Helligkeitswert, dem Balance-grenzwert, dem Drehpunktwert, dem Gradienten und dem Vorzeichen; und

Erzeugen der Maske basierend auf der Kontrastdehnungskurve.

12. Verfahren nach Anspruch 9, wobei der Schritt d) Folgendes aufweist:

Aufteilen des interessierenden Bereichs in eine Vielzahl von Bereichen, die eine bestimmte Größe aufweisen;

Berechnen eines durchschnittlichen Helligkeitswerts von jedem der Bereiche entsprechenden Pixeln;

Berechnen eines Drehpunktwerts der Kontrastdehnungskurve und eines Gradienten der Kontrastdehnungskurve basierend auf dem durchschnittlichen Helligkeitswert und dem Balancegrenzwert, wobei die Kontrastdehnungskurve Helligkeitsniveaus des Helligkeitsmodusbilds steuert;

Festlegen eines Vorzeichens der Kontrastdehnungskurve basierend auf dem Balancegrenzwert und dem Drehpunktwert;

Festlegen der Kontrastdehnungskurve basierend auf dem durchschnittlichen Helligkeitswert, dem Balance-grenzwert, dem Drehpunktwert, dem Gradienten und dem Vorzeichen; und

Erzeugen der Maske basierend auf der Kontrastdehnungskurve.

13. Verfahren nach Anspruch 12, wobei der Drehpunktwert aus der nachfolgend angegebenen Gleichung berechnet wird:

$$PPV = (V_{LM}+BTV)/2 \times V_{PPT}$$

wobei PPV der Drehpunktwert ist, $V_{LM}$ der durchschnittliche Helligkeitswert ist, BTV den Balancegrenzwert repräsentiert und $V_{PPT}$ den Drehpunktgrenzwert repräsentiert.

**14.** Verfahren nach einem der Ansprüche 8 bis 13, wobei der Schritt d) des Weiteren Folgendes aufweist:

Durchführen einer Vorverarbeitung an dem Helligkeitsmodusbild, um unnötige Bilddaten zu entfernen; und Durchführen eines morphologischen Prozesses an der Maske, um Leerräume aufzufüllen oder die Konnektivität an der Maske zu verbessern.

## Revendications

**1.** Système ultrasonique (100) comportant :

une unité d'acquisition de données ultrasoniques (120) configurée pour transmettre et recevoir des signaux ultrasoniques vers et en provenance d'un corps vivant pour acquérir des premières données ultrasoniques et des secondes données ultrasoniques ;
une unité d'entrée utilisateur (110) configurée pour recevoir des informations d'entrée correspondant à une zone d'intérêt et une valeur seuil sélectionnée correspondant à une brillance prédéterminée ; et
une unité de traitement (130) en communication avec l'unité d'acquisition de données ultrasoniques (120) et l'unité d'entrée utilisateur (110), l'unité de traitement (130) étant configurée pour former une image en mode brillance et une image couleurs en mode Doppler correspondant à la zone d'intérêt, basées respectivement sur les premières données ultrasoniques et les secondes données ultrasoniques, respectivement, pour déterminer une courbe de contraste d'étirement pour sécuriser la connectivité de vaisseaux sanguins du corps vivant, basé sur la valeur seuil sélectionnée de l'information d'entrée et de l'image en mode brillance, pour former un masque pour produire un processus de sélection sur l'image couleurs en mode Doppler, sur la base de la courbe de contraste de l'étirement, et pour réaliser le processus de sélection sur l'image couleurs en mode Doppler basée sur le masque.

**2.** Système ultrasonique (100) selon la revendication 1, dans lequel l'unité de traitement est configurée pour :

calculer une valeur de brillance moyenne des pixels correspondant à la zone d'intérêt de l'image en mode brillance ;
calculer une valeur de point de pivotement sur la courbe de contraste d'étirement et un gradient de la courbe de contraste d'étirement basé sur la valeur seuil sélectionnée, dans lequel la courbe de contraste d'étirement commande les niveaux de brillance de l'image en mode brillance,
déterminer un signe de la courbe de contraste d'étirement basé sur la valeur seuil sélectionnée et la valeur du point de pivotement ; et
déterminer la courbe de contraste d'étirement basé sur la valeur de brillance moyenne, la valeur seuil sélectionnée, la valeur du point de pivotement, le gradient et le signe.

**3.** Système ultrasonique (100) selon la revendication 2, dans lequel l'unité de traitement (130) est configurée pour calculer la valeur du point de pivotement selon l'équation suivante :

$$PPV = (V_{GB} + BTV)/2 \times V_{PPT}$$

dans laquelle PPV est la valeur du point de pivotement, $V_{GB}$ est la valeur de brillance moyenne, BTV est la valeur seuil sélectionnée, et $V_{PPT}$ est une valeur seuil du point de pivotement.

**4.** Système ultrasonique (100) selon la revendication 1, dans lequel l'unité de traitement (130) est configurée pour :

calculer une première valeur de brillance moyenne de pixels correspondant à la zone d'intérêt de l'image en mode brillance ;
diviser la zone d'intérêt en une pluralité de zones ayant une dimension prédéterminée ;
calculer une seconde valeur de brillance moyenne de pixels correspondant à chacune des zones ;
calculer une valeur de brillance moyenne de la première valeur de brillance moyenne et de la seconde valeur de brillance moyenne pour chacune des zones ;
calculer une valeur du point de pivotement de la courbe de contraste d'étirement et un gradient de la courbe de contraste d'étirement sur la base de la valeur moyenne de brillance et la valeur seuil sélectionnée, dans lequel la courbe de contraste d'étirement commande les niveaux de brillance de l'image en mode brillance ;
déterminer un signe de la courbe de contraste sur base de la valeur seuil sélectionnée et de la valeur du point de pivotement ; et
déterminer le contraste de la courbe de pivotement sur base de la valeur de brillance moyenne, de la valeur seuil sélectionnée, de la valeur du point de pivotement, du gradient et du signe.

5. Système ultrasonique (100) selon la revendication 1, dans lequel l'unité de traitement (130) est configurée pour :

diviser la zone d'intérêt en une pluralité de zones ayant une dimension prédéterminée ;
calculer une valeur de brillance moyenne de pixels correspondant à chacune des zones ;
calculer une valeur de point de pivotement de la courbe de contraste d'étirement et un gradient de la courbe de contraste d'étirement sur la base de la valeur moyenne de brillance et la valeur seuil sélectionnée, dans lequel la courbe de contraste d'étirement commande les niveaux de brillance de l'image en mode brillance ;
déterminer un signe de la courbe de contraste sur base de la valeur seuil sélectionnée et de la valeur du point de pivotement ; et
déterminer le contraste de la courbe de pivotement sur base de la valeur de brillance moyenne, de la valeur seuil sélectionnée, de la valeur du point de pivotement, du gradient et du signe.

6. Système ultrasonique (100) selon la revendication 5, dans lequel l'unité de traitement (130) est configurée pour calculer la valeur du point de pivotement selon l'équation suivante :

$$PPV = (V_{LM} + BTV)/2 \times V_{PPT}$$

dans laquelle PPV est la valeur du point de pivotement, $V_{LM}$ est la valeur de brillance moyenne, BTV représente la valeur seuil sélectionnée et $V_{PPT}$ représente la valeur moyenne du point de pivotement.

7. Système ultrasonique selon l'une quelconque des revendications 1 - 6, dans lequel l'unité de traitement (130) est configurée pour :

effectuer le traitement de l'image en mode brillance pour éliminer des données images inutiles ; et
effectuer un traitement morphologique sur le masque pour remplir des espaces vides ou accroître la connectivité sur le masque.

8. Procédé pour produire une image en couleurs en mode Doppler, comprenant :

a) former une image en mode brillance basée sur des premières données ultrasoniques pour un corps vivant ;
b) recevoir des informations d'entrée correspondant à une zone d'intérêt et une valeur seuil sélectionnée ayant une valeur de brillance prédéterminée d'un utilisateur ;
c) former une image couleurs en mode Doppler correspondant à la zone d'intérêt sur base de secondes données ultrasoniques pour le corps vivant ;
d) déterminer une courbe de contraste d'étirement pour sécuriser la connectivité de vaisseaux sanguins du corps vivant, sur la base de la valeur seuil sélectionnée des informations d'entrée et de l'image en mode brillance, et former un masque pour produire un processus seuil sur l'image couleurs en mode Doppler, sur la base de la courbe de contraste de l'étirement ; et
e) réaliser le processus seuil sur l'image couleurs en mode Doppler basée sur le masque.

9. Procédé selon la revendication 8, dans lequel l'étape d) consiste à :

calculer une valeur de brillance moyenne des pixels correspondant à la zone d'intérêt de l'image en mode brillance ;
calculer une valeur de point de pivotement sur la courbe de contraste d'étirement et un gradient de la courbe de contraste d'étirement basés sur la valeur moyenne de brillance et la valeur seuil sélectionnée, dans lequel la courbe de contraste d'étirement commande les niveaux de brillance de l'image en mode brillance ;

déterminer un signe de la courbe de contraste d'étirement sur base de la valeur seuil sélectionnée et de la valeur du point de pivotement ;
déterminer la courbe de contraste d'étirement pour former le masque basé sur la valeur moyenne de brillance, la valeur seuil sélectionnée, la valeur de point de pivotement, le gradient et le signe ; et
former le masque sur base de la courbe de contraste d'étirement.

**10.** Procédé selon la revendication 9, dans lequel la valeur du point de pivotement est calculée selon l'équation suivante :

$$PPV = (V_{GB} + BTV)/2 \times V_{PPT}$$

dans laquelle PPV est la valeur du point de pivotement, $V_{GB}$ est la valeur de brillance moyenne, BTV est la valeur seuil sélectionnée, et $V_{PPT}$ est une valeur seuil du point de pivotement.

**11.** Procédé selon la revendication 9, dans lequel l'étape d) comporte :

calculer une première valeur de brillance moyenne de pixels correspondant à la zone d'intérêt de l'image en mode brillance ;
diviser la zone d'intérêt en une pluralité de zones ayant une dimension prédéterminée ;
calculer une seconde valeur de brillance moyenne de pixels correspondant à chacune des zones ;
calculer une valeur de brillance moyenne de la première valeur de brillance des zones ;
calculer la valeur du point de pivotement de la courbe de contraste d'étirement et un gradient de la courbe de contraste d'étirement sur la base de la valeur moyenne de brillance et la valeur seuil sélectionnée, dans lequel la courbe de contraste d'étirement commande les niveaux de brillance de l'image en mode brillance ;
déterminer un signe de la courbe de contraste d'étirement sur base de la valeur seuil sélectionnée et de la valeur du point de pivotement ; et déterminer la courbe de contraste d'étirement pour former le masque sur base de la valeur de brillance moyenne, de la valeur seuil sélectionnée, de la valeur du point de pivotement, du gradient et du signe ; et
former le masque sur base de la courbe de contraste d'étirement.

**12.** Procédé selon la revendication 9, dans lequel l'étape d) comporte :

diviser la zone d'intérêt en une pluralité de zones ayant une dimension prédéterminée ;
calculer une valeur de brillance moyenne de pixels correspondant à chacune des zones ;
calculer la valeur du point de pivotement de la courbe de contraste d'étirement et un gradient de la courbe de contraste d'étirement sur la base de la valeur moyenne de brillance et la valeur seuil sélectionnée, dans lequel la courbe de contraste d'étirement commande les niveaux de brillance de l'image en mode brillance ;
déterminer un signe de la courbe de contraste d'étirement sur base de la valeur seuil sélectionnée et de la valeur du point de pivotement ;
déterminer la courbe de contraste d'étirement pour former le masque basé sur la valeur moyenne de brillance, la valeur seuil sélectionnée, la valeur de point de pivotement, le gradient et le signe ; et
former le masque sur base de la courbe de contraste d'étirement.

**13.** Procédé selon la revendication 12, dans lequel la valeur du point de pivotement est calculée selon l'équation suivante :

$$PPV = (V_{LM} + BTV)/2 \times V_{PPT}$$

dans laquelle PPV est la valeur du point de pivotement, $V_{LM}$ est la valeur de brillance moyenne, BTV représente la valeur seuil sélectionnée et $V_{PPT}$ représente la valeur moyenne du point de pivotement.

**14.** Procédé selon l'une quelconque des revendications 8 à 13, dans lequel l'étape d) consiste en outre à :

effectuer le traitement de l'image en mode brillance pour éliminer des données images inutiles ; et
effectuer un traitement morphologique sur le masque pour remplir des espaces vides ou accroître la connectivité

sur le masque.

# FIG. 1

# FIG. 2

# FIG. 3

120

Tx SIGNAL GENERATING SECTION
320

↓

ULTRASOUND PROBE
310

→

BEAM FORMER
330

→

ULTRASOUND DATA FORMING SECTION
340

# FIG. 4

START

↓

FORMING BRIGHTNESS MODE IMAGE — S402

↓

SETTING ROI — S404

↓

FORMING COLOR DOPPLER MODE IMAGE — S406

↓

FORMING BALANCE MASK — S408

↓

PERFORMING BALANCE PROCESS — S410

↓

FORMING COMPOUND IMAGE — S412

↓

END

# FIG. 5

START

PERFORMING  PREPROCESSING UPON BRIGHTNESS MODE IMAGE — S502

SETTING IMAGE PARAMETER — S504

FORMING BALANCE MASK — S506

PERFORMING MORPHOLOGICAL PROCESS — S508

END

# FIG. 6

## FIG. 7

## FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020100085969

- US 2005059892 A1

**Non-patent literature cited in the description**

- **JACINTA E BROWNE et al.** Colour Doppler Spatial Resolution Performance Testing. Ultrasound, Royal Society of Medicine Press, 01 August 2007, vol. 15, 162-166